# EUROPÄISCHE PATENTSCHRIFT

(11) **EP 3 743 050 B1**
(45) Veröffentlichungstag und Bekanntmachung des Hinweises auf die Patenterteilung: **29.03.2023**
(21) Anmeldenummer: 19702080.3
(22) Anmeldetag: 28.01.2019
(51) Int. Cl.: A61K 9/70, A61K 47/10, A61K 9/00, A61K 47/32, A61K 47/36

(54) **MEHRSCHICHTIGER ORALER DÜNNFILM**
MULTI-LAYER ORAL THIN FILM
FILM MINCE MULTICOUCHE D'HYGIÈNE BUCCO-DENTAIRE

(30) Priorität: 26.01.2018 DE 102018101778
(43) Veröffentlichungstag der Anmeldung: 02.12.2020
(73) Patentinhaber: LTS Lohmann Therapie-Systeme AG, 56626 Andernach (DE)
(72) Erfinder: SCHMITZ, Christoph, 56598 Rheinbrohl (DE); LINN, Michael, 55596 Waldböckelheim (DE)
(74) Vertreter: Held, Stephan
(86) Internationale Anmeldenummer: PCT/EP2019/051960
(87) Internationale Veröffentlichungsnummer: WO 2019/145524

(56) Entgegenhaltungen:
- US-A1- 2011 290 694

## Beschreibung

Die vorliegende Erfindung betrifft einen mehrschichtigen oralen Dünnfilm, ein Verfahren zu dessen Herstellung und dessen Verwendung als Arzneimittel.

Orale Dünnfilme sind dünne, einen pharmazeutisch aktiven Wirkstoff enthaltende Filme, die direkt in den Mundraum gelegt oder an die Mundschleimhaut angelegt werden und sich dort auflösen. Dabei handelt es sich insbesondere um dünne wirkstoffhaltige Filme auf Polymerbasis, die, wenn sie auf eine Schleimhaut, insbesondere die Mundschleimhaut, aufgebracht werden, den Wirkstoff direkt in diese abgeben. Diese oralen Dünnfilme sind in der Regel nach außen nicht klebrig. Die sehr gute Durchblutung der Mundschleimhaut sorgt für einen schnellen Übergang des Wirkstoffs in den Blutkreislauf. Dieses Darreichungssystem hat den Vorteil, dass der Wirkstoff größtenteils durch die Schleimhaut resorbiert wird und damit der "First-Pass Metabolismus", der bei der konventionellen Darreichungsform eines Wirkstoffs in Tablettenform, die in der Regel mit Flüssigkeit eingenommen werden, was nachteilig sein kann, auftritt, vermieden wird. Der Wirkstoff kann in dem Film gelöst, emulgiert oder dispergiert werden. Geeignete Wirkstoffe können nach Auflösen des oralen Dünnfilms im Mund auch abgeschluckt werden und somit über den Gastrointestinaltrakt aufgenommen werden.

Die aus dem Stand der Technik bekannten oralen Dünnfilme haben den Nachteil, dass das maximale Flächengewicht und damit die Menge an enthaltenen pharmazeutisch aktiven Wirkstoff durch die Trocknung des oralen Dünnfilms bei dessen Herstellung bestimmt wird. Je größer das Flächengewicht des oralen Dünnfilms ist, desto mehr pharmazeutisch aktiver Wirkstoff kann zwar darin enthalten sein, jedoch wird dadurch die Trockenzeit des oralen Dünnfilms auf eine nicht mehr wirtschaftliche Zeit verlängert. Diesem Nachteil kann zwar durch eine erhöhte Trockentemperatur entgegengewirkt werden, allerdings wird damit der pharmazeutisch aktive Wirkstoff einer unerwünschten thermischen Belastung ausgesetzt. Zudem weisen orale Dünnfilme mit einem hohen Flächengewicht eine relative lange Zerfallsdauer auf, was je nach Anwendung unerwünscht sein kann.

Die oben genannten Probleme können grundsätzlich durch mehrschichtige orale Dünnfilme überwunden werden.

Mehrschichtige orale Dünnfilme sind aus dem Stand der Technik bekannt.

So offenbart die WO 2011/134846 A1 mehrschichtige orale Dünnfilme, umfassend eine wirkstoffhaltige Schicht und eine Schicht, enthaltend eine Substanz, die inkompatibel mit dem Wirkstoff in der wirkstoffhaltigen Schicht ist, wobei diese beiden Schichten durch eine weitere zwischen diesen beiden Schichten liegende Schutzschicht getrennt sind.

Die US 2013/0017235 A1 offenbart mehrschichtige orale Dünnfilme, bei denen eine wirkstoffhaltige Schicht von zwei wasserquellbaren Polymerschichten eingeschlossen wird.

Die US 2011/290694 offenbart mehrschichtige orale Dünnfilme, die mindestens zwei gleich große Schichten umfassen, und die wasserlösliche Polymere sowie einen Arzneistoff enthalten, wobei die Schichten miteinander heiß versiegelt sind.

Die bekannten mehrschichtigen oralen Dünnfilme bei denen mehrere einzelne Schichten mit einem relativ geringen Flächengewicht zu einem Verbund verklebt oder laminiert werden, haben jedoch mehrere Nachteile. Zum einen müssen gegebenenfalls größere Mengen an geeigneten Klebstoffen zwischen den Schichten eingesetzt werden. Zum anderen können solche mehrschichtigen Systeme dadurch hergestellt wird, dass zunächst eine erste Schicht hergestellt wird und nach dem diese getrocknet wurde, eine zweite Schicht auf die erste Schicht laminiert wird. Nach dem Trocknen der zweiten Schicht auf der ersten Schicht kann dann gegebenenfalls eine dritte Schicht auflaminiert werden. Durch ein solches Verfahren können zwar vielschichtige Dünnfilme bereitgestellt werden, allerdings jeweils nur durch das Auflaminieren weiterer Schichten auf eine bereits bestehende Schicht. Dies wiederrum hat den Nachteil, dass der pharmazeutisch aktive Wirkstoff aufgrund mehrfacher Beschichtung stärker thermisch belastet wird. Zudem ist ein durch Auflaminieren entstandener Verbund, insbesondere wenn die einzelnen Schichten des oralen Dünnfilms nicht klebrig sind, oft nicht stabil und kann leicht zerfallen.

Die Aufgabe der vorliegenden Erfindung besteht darin, vorstehend genannte Nachteile des Standes der Technik zu beheben. Insbesondere besteht die Aufgabe der vorliegenden Erfindung darin, einen mehrschichtigen oralen Dünnfilm bereitzustellen, der aus mehreren einzelnen Schichten mit einem relativ geringen Flächengewicht aufgebaut ist, die untereinander fest verbunden sind, sodass die einzelnen Schichten in einer wirtschaftlich akzeptablen Zeit getrocknet werden können und der pharmazeutisch aktive Wirkstoff geringer thermisch belastet wird. Zudem soll sich der mehrschichtige orale Dünnfilm bei der Applikation in der Mundhöhle relativ schnell auflösen.

Obige Aufgabe wird durch einen mehrschichtigen oralen Dünnfilm nach Anspruch 1 gelöst, wobei der mehrschichtige orale Dünnfilm mindestens zwei übereinander angeordnete Schichten umfasst, die jeweils mindestens ein wasserlösliches Polymer umfassen, wobei diese mindestens zwei Schichten durch mindestens eine Siegelung miteinander verbunden sind, und wobei mindestens eine Siegelung nicht vollflächig ist.

Ein solcher mehrschichtiger oraler Dünnfilm zeichnet sich dadurch aus, dass durch den Einsatz mehrerer Schichten die Menge an pharmazeutisch aktivem Wirkstoff, bezogen auf den gesamten oralen Dünnfilm, erhöht werden kann, ohne dass lange Trocknungszeiten erforderlich sind oder eine thermische Belastung des pharmazeutisch aktiven Wirkstoffs in Kauf genommen werden muss. Insbesondere kann so möglichst weitgehend verhindert werden, dass der orale Dünnfilm zum Trocknen über eine längere Zeit, insbesondere länger als etwa 20 min, Temperaturen, wie sie üblicherweise zum Trocknen oraler Dünnfilme eingesetzt werden, insbesondere Temperaturen größer etwa 70°C, ausgesetzt wird.

Insbesondere weist der erfindungsgemäße mehrschichtige orale Dünnfilm durch die nicht vollflächige Siegelung eine deutlich größere Oberfläche im Vergleich zu herkömmlichen oralen Dünnfilmen auf. Durch die größere Oberfläche löst sich der erfindungsgemäße mehrschichtige orale Dünnfilm bei Applikation in der Mundhöhle deutlich schneller auf. Insbesondere durch die nicht vollflächige Siegelung kann der Speichel zwischen die einzelnen Schichten eindringen und damit zu einer schnelleren Auflösung des oralen Dünnfilms führen.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung dafür ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Unter Siegelung wird jede mögliche Art und Weise verstanden, durch die mindestens zwei Schichten des mehrschichtigen oralen Dünnfilms, wobei jede Schicht mindestens ein wasserlösliches Polymer umfasst, miteinander verbunden werden können. Dies umfasst beispielsweise aber nicht abschließend Verbindungen durch Verklebung, Prägung, Laminierung und/oder Heißsiegelung.

Unter einer nicht vollflächigen Siegelung wird verstanden, dass die Fläche durch die die mindestens zwei Schichten des mehrschichtigen oralen Dünnfilms miteinander verbunden sind kleiner ist als die Oberfläche einer Seite einer Schicht des mehrschichtigen oralen Dünnfilms. Diese nicht vollflächige Siegelung kann in Form von einem oder mehreren durchgehenden Streifen über die Oberfläche des mehrschichtigen oralen Dünnfilms erfolgen oder durch Siegelung an einem oder mehreren separierten Punkten.

Die mindestens zwei Schichten, die jeweils mindestens ein wasserlösliches Polymer umfassen können dieselbe oder eine verschiedene Zusammensetzung aufweisen. Auch können die mindestens zwei Schichten, die jeweils mindestens ein wasserlösliches Polymer umfassen, dieselbe oder eine verschiedene Größe bzw. Fläche aufweisen. Zudem können die mindestens zwei Schichten, die mindestens ein wasserlösliches Polymer umfassen, dasselbe oder ein verschiedenes Flächengewicht aufweisen.

Gemäß der Erfindung ist mindestens eine der überlappenden Kanten der mindestens zwei übereinander angeordneten Schichten nicht durch eine Siegelung verschlossen.

Dies hat den Vorteil, dass Speichel leicht von außen zwischen die einzelnen Schichten eindringen kann, was die Auflösungszeit des erfindungsgemäßen mehrschichtigen oralen Dünnfilms erhöht.

Die mindestens zwei Schichten des erfindungsgemäßen oralen Dünnfilms weisen die gleiche Größe bzw. Fläche auf.

Wenn die mindestens zwei Schichten des erfindungsgemäßen oralen Dünnfilms die gleiche Größe bzw. Fläche aufweisen, so ist es bevorzugt, dass die mindestens zwei Schichten deckungsgleich übereinander angeordnet sind, sodass die Kanten der mindestens zwei Schichten überlappen und keine der mindestens zwei Schichten über die andere hinausragt.

Es sind jedoch auch Ausführungsformen des erfindungsgemäßen oralen Dünnfilms denkbar, wobei die mindestens zwei Schichten gegeneinander verschoben sind, sodass diese nicht deckungsgleich aufeinander angeordnet sind.

Weiterhin ist der erfindungsgemäße mehrschichtige orale Dünnfilm dadurch gekennzeichnet, dass die mindestens eine nicht vollflächige Siegelung eine nicht vollflächige Heißsiegelung umfasst.

Der erfindungsgemäße mehrschichtige orale Dünnfilm ist daher bevorzugt dadurch gekennzeichnet, dass das mindestens eine Polymer ein heißsiegelbares Polymer umfasst.

Unter Heißsiegelung wird eine Verbindung der übereinanderliegenden mindestens zwei Schichten des mehrschichtige oralen Dünnfilms durch eine punktuelle Erwärmung und Anpressung der übereinanderliegenden mindestens zwei Schichten verstanden. Durch die punktuelle Erwärmung schmilzt das mindestens eine Polymer, das jeweils in den einzelnen Schichten des mehrschichtigen oralen Dünnfilms vorhanden ist, was nach dem erneuten Erkalten zu einer festen Verbindung der mindestens zwei Schichten führt. Bevorzugt wird dabei punktuell auf eine Temperatur erwärmt, die oberhalb der Schmelztemperatur bzw. Glasübergangstemperatur des jeweiligen Polymers liegt.

Übliche Temperaturen für eine Heißsiegelung betragen etwa 50°C bis 200°C.

Bei der Heißsiegelung wird der orale Dünnfilm vorzugsweise für etwa 5 Sekunden, bevorzugt etwa 3 Sekunden und besonders bevorzugt etwa 2 Sekunden oder weniger einer Temperatur von etwa 50°C bis 200°C.

Durch diese sehr kurzen Zeiten, die für die Heißsiegelung benötigt werden, ist die thermische Belastung des mindestens einen pharmazeutisch aktiven Wirkstoffs weniger stark als dies bei längeren Trocknungstemperaturen wie obenstehend beschrieben.

Unter einem heißsiegelbaren Polymer wird demnach ein Polymer verstanden, das durch punktuelle Erwärmung geschmolzen oder erweicht werden und damit eine Verbindung zu darüber oder darunterliegenden Schichten hergestellt werden kann.

Bevorzugt umfasst das mindestens eine wasserlösliche Polymer Polyvinylalkohol, Pullulan, Polyethylenoxid und/oder Polyethylenglykol, und Copolymere davon.

Diese Polymere haben den Vorteil, dass sie mit einer Vielzahl von pharmazeutisch aktiven Wirkstoffen kompatibel und zudem weitgehend unbedenklich für einen Patienten sind, zu dessen Behandlung der erfindungsgemäße mehrschichtige oralen Dünnfilm eingesetzt wird.

Zudem sind die aufgeführten Polymere bevorzugt, da sie heißsiegelbar sind.

Der erfindungsgemäße mehrschichtige orale Dünnfilm ist außerdem dadurch gekennzeichnet, dass mindestens eine Schicht des mehrschichtigen oralen Dünnfilms mindestens einen pharmazeutischen Wirkstoff umfasst.

Der pharmazeutisch aktive Wirkstoff kann in dem erfindungsgemäßen mehrschichtigen oralen Dünnfilm grundsätzlich in jeder der Schichten des erfindungsgemäßen mehrschichtigen oralen Dünnfilms enthalten sein, wobei die Kompatibilität des jeweiligen Wirkstoffs mit dem Material aus dem die jeweilige Schicht besteht und gegebenenfalls auch mit dem Material aus dem die anderen Schichten bestehen zu berücksichtigen.

Der erfindungsgemäße mehrschichtige orale oralen Dünnfilm ist nicht darauf beschränkt, dass nur ein pharmazeutisch aktiver Wirkstoff enthalten ist. So sind mehrschichtige orale Dünnfilme denkbar, bei denen in verschiedenen Schichten verschiedene pharmazeutisch aktive Wirkstoffe enthalten sind. Eine einzelne Schicht kann auch mehr als einen pharmazeutisch aktiven Wirkstoff enthalten.

Die vorliegende Erfindung ist besonders vorteilhaft bezüglich eines mehrschichtigen oralen Dünnfilms, der in verschiedenen Schichten verschiedene Wirkstoffe enthält. Erfindungsgemäß können diese verschiedenen Schichten alle separat hergestellt werden und dann gemäß eines Baukastenprinzips durch nicht vollflächige Siegelung miteinander verbunden werden.

Generell kann in dem erfindungsgemäßen mehrschichtigen oralen Dünnfilm jeder pharmazeutisch aktive Wirkstoff enthalten sein, der zur transmukosalen oder oralen Verabreichung geeignet ist.

Bevorzugt handelt es sich bei dem mindestens einen pharmazeutisch aktiven Wirkstoff um einen pharmazeutisch aktiven Wirkstoff, der ausgewählt ist aus der Gruppe, bestehend aus pharmazeutisch aktiven Wirkstoffen, die im Kontext der vorliegenden Erfindung für orale Applikationen geeignet sind. Dies sind beispielsweise antiallergische Mittel, anti-arrhythmische Mittel, Antibiotika, antidiabetische Mittel, anti-epileptische Mittel, Antihistaminika, Antitussiva, cardiotronische Mittel, Diuretika, blutdrucksenkende Mittel, Betäubungsmittel, Nervenmuskelblocker und Sexualhormone, wie Vasopressoren. Spezifische Beispiele sind Acetaminophen, Adrenalin, Alprazolam, Amlodipin, Anastrozol, Apomorphin, Aripiprazol, Atorvastatin, Baclofen, Benzocain, Benzocain/Menthol, Benzydamin, Buprenorphin, Buprenorphin/Naloxon, Buprenorphin/Naloxon/Cetirizin, Cetirizin, Chlorpheniramin, Clomipramin, Dexamethason, Dextromethorphan, Dextromethorphan/Phenylephrin, Diclofenac, Diphenhydramin, Diphenhydramin/Phenylephrin, Donepezil Dronabinol, Epinephrin, Escitalopram, Famotidin, Fentanyl, Glimepirid, GLP-1 Peptiden, Granisetron, Insulin, Insulin Nanopartikel, Insulin/GLP-1 Nanopartikel, Ketoprofen, Ketotifen, Koffein, Levocetirizin, Loperamid, Loratadin, Medizin, Methylphenidat, Midazolam, Mirodenafil, Montelukast, Multimeric-001, Naloxon, Nikotin, Nitroglycerin, Olanzapin, Olopatadin, Ondansetron, Oxybutynin, Pektin, Pektin/Menthol, Pectin/Ascorbinsäure, PediaSUNAT (Artesunat und Amodiaquin), Piroxicam, Phenylephrin, Prednisolon, Pseudoephedrin, Risperidon, Rivastigmin, Rizatriptan, Selegiline, Senna Glykosiden, Sildenafil Zitrat, Simethicon, Sumatriptan, Tadalafil, Testosteron, Triamcinolon Azetonid, Triptan, Tropicamide, Voglibose, Zolmitriptan, Zolpidem, oder pharmazeutisch akzeptable Salze dieser Verbindungen. Als nicht pharmazeutische Wirkstoffe kann die erfindungsgemäße Verabreichungsform z.B. Wirkstoffe für die Mundhygiene, wie Menthol, enthalten. Der pharmazeutische Wirkstoff kann auch eine Mischung von unterschiedlichen Wirkstoffen sein.

Der mindestens eine pharmazeutisch aktive Wirkstoff ist grundsätzlich mindestens in einer pharmazeutisch wirksamen Menge in mindestens einer der Schichten des erfindungsgemäßen mehrschichtigen oralen Dünnfilms enthalten.

Bevorzugt ist es, wenn der pharmazeutisch aktive Wirkstoff in mindestens einer Schicht des mehrschichtigen oralen Dünnfilms in einer Menge von 1 bis 60 Gew.- % vorliegt.

Grundsätzlich ist die Anzahl der einzelnen Siegelungen zur Verbindung der einzelnen Schichten des erfindungsgemäßen mehrschichtigen oralen Dünnfilms nicht begrenzt, solange die Summe der einzelnen Flächen der Siegelungen kleiner ist als die Fläche einer Seite des erfindungsgemäßen mehrschichtigen oralen Dünnfilms.

Es ist bevorzugt, dass die mindestens zwei Schichten durch genau eine nicht vollflächige

Siegelung miteinander verbunden sind.

Außerdem ist es bevorzugt, wenn der erfindungsgemäße orale Dünnfilm dadurch gekennzeichnet ist, dass die mindestens zwei Schichten durch zwei nicht vollflächige Siegelungen miteinander verbunden sind.

Der erfindungsgemäße mehrschichtige orale Dünnfilm zeichnet sich bevorzugt dadurch aus, dass die mindestens eine Siegelung nicht mehr als etwa 66% der gesamten Fläche einer Oberfläche des mehrschichtigen oralen Dünnfilms umfasst.

Die Form der mindestens einen nicht vollflächigen Siegelung ist nicht beschränkt.

Zweckmäßigerweise wird die Siegelung in Streifenform und/oder Punktform durchgeführt.

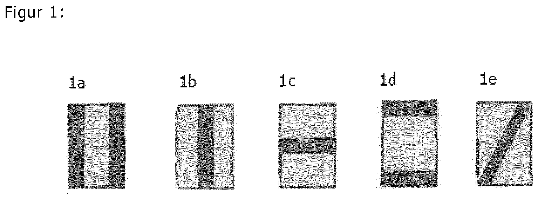

Schematische Darstellungen erfindungsgemäßer mehrschichtiger oraler Dünnfilme, wobei die einzelnen Schichten durch eine oder zwei nicht vollflächige Siegelungen miteinander verbunden sind, sind in den Figuren 1a bis 1e abgebildet.

Der erfindungsgemäße mehrschichtige orale Dünnfilm ist weiterhin vorzugsweise dadurch gekennzeichnet, dass die mindestens zwei nicht-klebenden Schichten jeweils ein Flächengewicht von 20 bis 300 g/m², aufweisen.

Ist das Flächengewicht höher, so hat das den Nachteil besonders langer Trocknungszeiten, was wirtschaftlich unvorteilhaft ist. Zudem kann es zu Blasenbildung durch Hautbildung kommen. Ein Film mit einem geringeren Flächengewicht lässt sich nur schwer verarbeiten.

In den jeweiligen Schichten des erfindungsgemäßen mehrschichtigen oralen Dünnfilms können außerdem übliche Zusatzstoffe, wie Permeationserhöher, Antioxidantien, Geschmacksstoffe, geschmacksmaskierende Stoffe, Konservierungsstoffe, Farbstoffe, inerte Füllstoffe etc., enthalten sein.

Die vorliegende Erfindung betrifft auch ein Verfahren zur Herstellung eines mehrschichtigen oralen Dünnfilms, wie vorstehend definiert, umfassend die Schritte
a) Bereitstellen einer ersten Schicht umfassend mindestens ein wasserlösliches Polymer;
b) Bereitstellen einer weiteren Schicht umfassend mindestens ein wasserlösliches Polymer;
c) Anordnen der ersten Schicht auf der weiteren Schicht; und
d) Ausführen mindestens einer nicht vollflächigen Siegelung, um die erste Schicht und die weitere Schicht miteinander zu verbinden.

Es sind Ausführungsformen denkbar, bei denen die erste und die weitere Schicht die gleiche Zusammensetzung aufweisen. Ebenso sind Ausführungsformen denkbar, bei denen die erste und die weitere Schicht verschiedene Zusammensetzungen aufweisen.

In dem erfindungsgemäßen Verfahren umfasst mindestens eine der in Schritt a) und/oder Schritt b) bereitgestellten Schichten außerdem mindestens einen pharmazeutischen Wirkstoff.

Außerdem ist das erfindungsgemäße Verfahren vorteilhafterweise dadurch gekennzeichnet, dass die mindestens eine in Schritt d) ausgeführte nicht vollflächige Siegelung eine nicht vollflächige Heißsiegelung ist.

In einer mögliche Ausführungsform weist die ersten Schicht (a) die selbe Zusammensetzung auf wie die weitere Schicht (b).

In einer weiteren möglichen Ausführungsform weist die ersten Schicht a) eine andere Zusammensetzung auf wie die weitere Schicht (b).

In einer möglichen Ausführungsform umfasst die erste Schicht a) und/oder die weitere Schicht b) bereits mehr als eine Schicht. Damit sind mehrschichtige orale Dünnfilme erhältlich, die mindestens drei Schichten umfassen.

Prinzipiell können mit dem erfindungsgemäßen Verfahren somit mehrschichtige orale Dünnfilme bereitgestellt werden, die über mindestens zwei aber auch beliebig viele Schichten verfügen.

Die vorliegende Erfindung betrifft außerdem einen mehrschichtigen oralen Dünnfilm, erhältlich nach dem vorstehend geschilderten Verfahren.

Die vorliegende Erfindung betrifft weiterhin einen mehrschichtigen oralen Dünnfilm, wie vorstehend beschrieben oder wie nach dem vorstehend geschilderten Verfahren erhältlich, zur Verwendung als Arzneimittel.

Die Erfindung wird nachfolgend anhand von nicht beschränkenden Beispielen erläutert.

### Beispiele:

Schematische Darstellung möglicher erfindungsgemäßer mehrschichtiger oraler Dünnfilme, wobei die jeweilige(n) nicht vollflächige(n) Siegelung(en), die zur Verbindung der einzelnen Schichten in dem erfindungsgemäßen mehrschichtigen oralen Dünnfilm eingesetzt wurde(n), durch den dunkleren Farbton schematisch hervorgehoben ist/sind, sind in den Figuren 1a bis 1e dargestellt.

## Patentansprüche

1. Mehrschichtiger oraler Dünnfilm, umfassend mindestens zwei gleich große übereinander angeordnete Schichten, die jeweils mindestens ein wasserlösliches Polymer umfassen und wobei mindestens eine Schicht mindestens einen pharmazeutischen Wirkstoff umfasst, wobei diese mindestens zwei Schichten durch mindestens eine Siegelung miteinander verbunden sind, **dadurch gekennzeichnet, dass** die mindestens eine Siegelung nicht vollflächig ist und wobei die mindestens eine Siegelung derart angebracht ist, dass mindestens eine überlappende Kante der mindestens zwei übereinander angeordneten Schichten nicht durch eine Siegelung verschlossen ist.

2. Mehrschichtiger oraler Dünnfilm gemäß einem der vorhergehenden Ansprüche, **dadurch gekennzeichnet, dass** das mindestens eine wasserlösliche Polymer ein heißsiegelbares Polymer umfasst.

3. Mehrschichtiger oraler Dünnfilm gemäß dem vorherigen Anspruch 2, **dadurch gekennzeichnet, dass** die mindestens eine nicht vollflächige Siegelung eine nicht vollflächige Heißsiegelung umfasst.

4. Mehrschichtiger oraler Dünnfilm gemäß einem der vorhergehenden Ansprüche, **dadurch gekennzeichnet, dass** das mindestens eine wasserlösliche Polymer Polyvinylalkohol, Pullulan, Polyethylenoxid, Polyethylenglykol und/oder Copolymere davon umfasst.

5. Mehrschichtiger oraler Dünnfilm gemäß einem der vorhergehenden Ansprüche, **dadurch gekennzeichnet, dass** die mindestens zwei Schichten durch genau eine nicht vollflächige Siegelung miteinander verbunden sind.

6. Mehrschichtiger oraler Dünnfilm gemäß einem der Ansprüche 1-4, **dadurch gekennzeichnet, dass** die mindestens zwei Schichten durch zwei nicht vollflächige Siegelungen miteinander verbunden sind.

7. Mehrschichtiger oraler Dünnfilm gemäß einem der vorhergehenden Ansprüche, **dadurch gekennzeichnet, dass** die mindestens eine Siegelung nicht mehr als 66% der gesamten Fläche einer Oberfläche des mehrschichtigen oralen Dünnfilms umfasst.

8. Verfahren zur Herstellung eines mehrschichtigen oralen Dünnfilms, gemäß einem der Ansprüche 1 bis 7 umfassend die Schritte:
a) Bereitstellen einer ersten Schicht umfassend mindestens ein wasserlösliches Polymer;
b) Bereitstellen einer weiteren Schicht umfassend mindestens ein wasserlösliches Polymer, die die gleiche Größe wie die erste Schicht aufweist, wobei mindestens eine der in Schritt a) und/oder Schritt b) bereitgestellten Schichten außerdem mindestens einen pharmazeutischen Wirkstoff umfasst;
c) Anordnen der ersten Schicht auf der weiteren Schicht; und
d) Ausführen mindestens einer nicht vollflächigen Siegelung, um die erste Schicht und die weitere Schicht miteinander zu verbinden, wobei die mindestens eine Siegelung derart angebracht ist, dass mindestens eine überlappende Kante der mindestens zwei übereinander angeordneten Schichten nicht durch eine Siegelung verschlossen ist.

9. Verfahren zur Herstellung eines mehrschichtigen oralen Dünnfilms, gemäß Anspruch 8, **dadurch gekennzeichnet, dass** die mindestens eine in Schritt d) ausgeführte nicht vollflächige Siegelung eine nicht vollflächige Heißsiegelung ist.

10. Mehrschichtiger oraler Dünnfilm erhältlich gemäß dem Verfahren gemäß einem der Ansprüche 8 oder 9.

11. Mehrschichtiger oraler Dünnfilm gemäß einem der Ansprüche 1 bis 7 oder 10 zur Verwendung als Arzneimittel.

## Claims

1. A multilayer oral thin film comprising at least two equally sized layers arranged on top of each other, each comprising at least one water-soluble polymer and wherein at least one layer comprises at least one pharmaceutically active agent, said at least two layers being joined together by at least one seal, **characterized in that** said at least one seal is not full surface and wherein said at least one seal is mounted such that at least one overlapping edge of said at least two layers arranged on top of each other is not closed by a seal.

2. The multilayer oral thin film according to any one of the preceding claims, **characterized in that** the at least one water-soluble polymer comprises a heat-sealable polymer.

3. The multilayer oral thin film according to the preceding claim 2, **characterized in that** said at least one non-full surface seal comprises a non-full surface heat seal.

4. The multilayer oral thin film according to any one of the preceding claims, **characterized in that** the at least one water-soluble polymer comprises polyvinyl alcohol, pullulan, polyethylene oxide, polyethylene glycol and/or copolymers thereof.

5. The multilayer oral thin film according to any one of the preceding claims, **characterized in that** said at least two layers are joined together by exactly one non-full surface seal.

6. The multilayer oral thin film according to any one of claims 1-4, **characterized in that** the at least two layers are joined together by two non-full surface seals.

7. The multilayer oral thin film according to any one of the preceding claims, **characterized in that** said at least one seal comprises not more than 66% of the total area of a surface of the multilayer oral thin film.

8. A method of preparing a multilayer oral thin film according to any one of claims 1 to 7 comprising the steps of:
a) providing a first layer comprising at least one water-soluble polymer;
b) providing a further layer comprising at least one water-soluble polymer, which has the same size as the first layer, wherein at least one of the layers provided in step a) and/or step b) further comprises at least one pharmaceutically active agent;
c) placing the first layer on the further layer; and
d) performing at least one non-full surface sealing in order to join the first layer and the further layer to one another, wherein the at least one sealing is mounted in such a way that at least one overlapping edge of said at least two layers arranged on top of each other is not closed by a sealing.

9. The process for the preparation of a multilayer oral thin film according to claim 8, **characterized in that** the at least one non-full surface sealing performed in step d) is a non-full surface heat sealing.

10. A multilayer oral thin film obtainable according to the method of any one of claims 8 or 9.

11. The multilayer oral thin film according to any one of claims 1 to 7 or 10 for use as a medicament.

## Revendications

1. Film mince multicouche d'hygiène bucco-dentaire, comprenant au moins deux couches superposées de même dimension, qui comprennent respectivement au moins un polymère hydrosoluble et dans lequel au moins une couche comprend au moins un principe actif pharmaceutique, dans lequel ces au moins deux couches sont reliées l'une à l'autre par au moins un scellement, **caractérisé en ce que** le au moins un scellement n'est pas présent sur toute la surface et dans lequel le au moins un scellement est appliqué de telle sorte qu'au moins un bord chevauchant des au moins deux couches superposées n'est pas fermé par un scellement.

2. Film mince multicouche d'hygiène bucco-dentaire selon l'une quelconque des revendications précédentes, **caractérisé en ce que** le au moins un polymère hydrosoluble comprend un polymère thermoscellable.

3. Film mince multicouche d'hygiène bucco-dentaire selon la revendication 2 précédente, **caractérisé en ce que** le au moins un scellement non présent sur toute la surface comprend un thermoscellement non présent sur toute la surface.

4. Film mince multicouche d'hygiène bucco-dentaire selon l'une quelconque des revendications précédentes, **caractérisé en ce que** le au moins un polymère hydrosoluble comprend le polymère alcool polyvinylique, le pullulane, l'oxyde de polyéthylène, le polyéthylène glycol et/ou des copolymères de ceux-ci.

5. Film mince multicouche d'hygiène bucco-dentaire selon l'une quelconque des revendications précédentes, **caractérisé en ce que** les au moins deux couches sont reliées l'une à l'autre par exactement un seul scellement non présent sur toute la surface.

6. Film mince multicouche d'hygiène bucco-dentaire selon l'une quelconque des revendications 1-4, **caractérisé en ce que** les au moins deux couches sont reliées l'une à l'autre par deux scellements non présents sur toute la surface.

7. Film mince multicouche d'hygiène bucco-dentaire selon l'une quelconque des revendications précédentes, **caractérisé en ce que** le au moins un scellement n'englobe pas plus de 66 % de la superficie totale d'une surface du film mince multicouche d'hygiène bucco-dentaire.

8. Procédé pour la fabrication d'un film mince multicouche d'hygiène bucco-dentaire, selon l'une quelconque des revendications 1 à 7 comprenant les étapes de :
a) fourniture d'une première couche comprenant au moins un polymère hydrosoluble ;
b) fourniture d'une autre couche comprenant au moins un polymère hydrosoluble, qui présente la même dimension que la première couche, dans lequel au moins une des couches fournies à l'étape a) et/ou l'étape b) comprend en outre au moins un principe actif pharmaceutique ;
c) disposition de la première couche sur l'autre couche ; et
d) réalisation d'au moins un scellement non présent sur toute la surface, afin de relier la première couche et l'autre couche l'autre à l'autre, dans lequel le au moins un scellement est appliqué de telle sorte qu'au moins un bord chevauchant des au moins deux couches superposées n'est pas fermé par un scellement.

9. Procédé pour la fabrication d'un film mince multicouche d'hygiène bucco-dentaire, selon la revendication 8, **caractérisé en ce que** le au moins un scellement non présent sur toute la surface réalisé à l'étape d) est un thermoscellement non présent sur toute la surface.

10. Film mince multicouche d'hygiène bucco-dentaire pouvant être obtenu selon le procédé selon l'une quelconque des revendications 8 ou 9.

11. Film mince multicouche d'hygiène bucco-dentaire selon l'une quelconque des revendications 1 à 7 ou 10 pour son utilisation comme médicament.
